# EUROPÄISCHE PATENTSCHRIFT

(11) **EP 3 691 577 B1**
(45) Veröffentlichungstag und Bekanntmachung des Hinweises auf die Patenterteilung: **14.01.2026**
(21) Anmeldenummer: 18773396.9
(22) Anmeldetag: 17.09.2018
(51) Int. Cl.: A61F 5/01

(54) **GELENK FÜR EINE ORTHOPÄDIETECHNISCHE EINRICHTUNG**
ARTICULATION FOR AN ORTHOPEDIC DEVICE
ARTICULATION POUR UN DISPOSITIF ORTHOPEDIQUE

(30) Priorität: 04.10.2017 DE 102017122997
(43) Veröffentlichungstag der Anmeldung: 12.08.2020
(73) Patentinhaber: Ottobock SE & Co. KGaA, 37115 Duderstadt (DE)
(72) Erfinder: LÜRSSEN, Marcus, 37073 Göttingen (DE); SCHILLING, Matthias, 37345 Weißenborn-Lüderode (DE); MÜLLER, André, 37115 Duderstadt (DE)
(74) Vertreter: Gramm, Lins & Partner Patent- und Rechtsanwälte PartGmbB
(86) Internationale Anmeldenummer: PCT/EP2018/075026
(87) Internationale Veröffentlichungsnummer: WO 2019/068450

(56) Entgegenhaltungen:
- WO-A1-93/09734
- DE-A1- 102015 112 283
- US-A- 5 395 304
- US-A1- 2016 361 189

## Beschreibung

Die Erfindung betrifft ein Gelenk für eine orthopädietechnische Einrichtung, wobei das Gelenk ein erstes Element, wenigstens ein elastisches Element an einem Träger, der an dem ersten Element montiert ist, und ein zweites Element aufweist, das an dem ersten Element schwenkbar angeordnet ist und in einer Richtung ab einem ersten Eingriffswinkel zwischen dem ersten Element und dem zweiten Element gegen eine von dem wenigstens einen elastischen Element aufgebrachte Kraft verschwenkbar ist. Die Erfindung betrifft zudem einen Träger für ein derartiges Gelenk.

Ein derartiges Gelenk ist beispielsweise in Form eines Knöchelgelenkes für eine Beinorthese aus der DE 10 2010 014 334 A1 oder der DE 10 2013 011 382 A1 bekannt. Derartige Knöchelgelenke können bei Bein- oder Unterschenkelorthesen verwendet werden. Dabei kann es aus therapeutischen Gründen sinnvoll sein, die Länge der Schwenkbewegung, also den maximal möglichen Schwenkwinkel des zweiten Elementes relativ zum ersten Element zu begrenzen und beispielsweise in einer oder beiden Schwenkrichtungen jeweils einen Anschlag vorzusehen. Um ein zu hartes Anschlagen an diesen Anschlägen zu vermeiden, sind diese Anschläge in der Regel federbelastet und damit gedämpft ausgeführt. Diese Federung sorgt zudem dafür, dass ein Verschwenken des Gelenkes für die orthopädietechnische Einrichtung nur möglich ist, wenn die durch die Feder aufgebrachte Kraft überwunden wird. Auch dies kann zu Rehabilitations- und Trainingszwecken sinnvoll sein.

Aus der WO 2016/201318 A1 ist es bekannt, zur Einstellung der Nullstellung eines Knöchelgelenkes, also des Winkels zwischen dem Unterschenkelanteil und dem Fußteil, bei dem sich das Kräftegleichgewicht einstellt, dadurch zu verändern, dass der eigentliche Unterschenkel in Form einer Schiene relativ zu dem ersten Element des Gelenks verstellbar ist. Dokument US 2016/361189 A1 zeigt ebenfalls ein Gelenk für eine orthopädietechnische Einrichtung.

Bei derartigen Gelenken ist es oftmals von Vorteil, wenn beim Verschwenken des zweiten Elementes relativ zum ersten Element in die eine Richtung nicht über den gesamten Verschwenkweg eine Kraft aufgebracht werden muss, durch die die von dem elastischen Element aufgebrachte Kraft überwunden wird. Es kann durchaus von Vorteil sein, wenn bis zum Erreichen eines ersten Eingriffswinkels keine Federkraft überwunden werden muss und erst bei einem weiteren Verschwenken die Feder beispielsweise gestaucht wird, so dass sie eine entgegengesetzte Kraft aufbringt.

Als elastisches Element kann insbesondere ein elastisches Element oder eine Mehrzahl von elastischen Elementen verwendet werden. Verwendbare elastische Elemente sind beispielsweise Schraubenfedern, Tellerfedern oder Stapel von Tellerfedern oder Schraubentellerfedern. Andere elastische Elemente sind beispielsweise gummielastische Elemente wie Elastomerblöcke. Alle diese Elemente können mit einer Vorspannung versehen werden, die vorzugsweise einstellbar ist. Im Folgenden werden sofern von einem elastischen Element gesprochen wird, auch andere elastische Elemente gemeint.

Insbesondere bei der Verwendung des Gelenkes als Knöchelgelenk aber auch bei anderen Anwendungsfeldern muss dabei das elastische Element eine ausreichend große Federkraft und Federkonstante aufweisen und gleichzeitig möglichst wenig Bauraum benötigen. Aus dem Stand der Technik ist daher bekannt, einen Stapel von übereinander angeordneten Tellerfederelementen zu verwenden. Diese weisen eine hohe Federkraft und relativ geringen Bauraum auf. Aus der DE 10 2015 112 238 A1 ist bekannt, statt einer derartigen Anordnung mehrerer Tellerfedern eine Schraubentellerfeder zu verwenden. Diese hat insbesondere bei einem Bruch der Feder eine deutliche höhere Sicherheit als der aus dem Stand der Technik bekannte Tellerfederstapel.

Der erste Eingriffswinkel ist in der Regel dann erreicht, wenn ein Anlageelement des Trägers mit einer entsprechenden Anlagefläche des zweiten Elementes in Kontakt kommt, so dass ein weiteres Verschwenken des zweiten Elementes relativ zum ersten Element zwangsläufig zu einem Spannen der Feder, beispielsweise einem Komprimieren einer Druckfeder, führt. Herkömmlicherweise ist das Anlageelement beispielsweise eine Schraube, die in das dem zweiten Element abgewandte Ende des Trägers eingeschraubt wird und deren Ende die Anschlagsfläche bildet. Je nachdem, wie weit die Schraube oder das Anlageelement in den Träger eingeschraubt wird, lässt sich dadurch die Position des ersten Eingriffswinkels festlegen und einstellen. Nachteilig ist jedoch, dass dazu das Gelenk demontiert werden muss.

Durch die Einstellbarkeit des Anlageelementes wird der erste Eingriffswinkel eingestellt. Oftmals verfügt ein entsprechendes Gelenk über zwei vorzugsweise identisch ausgebildete Träger mit elastischen Elementen, die jeweils ein Anlageelement aufweisen, das auf die genannte Weise einstellbar ist. Dadurch ist es möglich, eine "Null-Stellung" des Gelenkes einzustellen. Unter der "Null-Stellung" wird dabei die Stellung verstanden, in der sich das erste Element relativ zum zweiten Element befindet, wenn keine äußeren Kräfte auf das Gelenk wirken und die Position der beiden Elemente zumindest nahezu vollständig, bevorzugt jedoch vollständig durch die durch die elastischen Elemente aufgebrachten Kräfte bestimmt wird. Dies ist beispielsweise von Vorteil, um eine Knöchelorthese oder Beinorthese an eine geänderte Absatzhöhe eines Schuhs anzupassen.

Aus dem Stand der Technik ist ein Gelenk bekannt, bei dem diese "Null-Stellung" durch die beiden Kräfte der auf das zweite Gelenkteil wirkenden elastischen Elemente bestimmt wird. Dies hat den Nachteil, dass eine Vorspannung der elastischen Elemente zwangsläufig eine Änderung der "Null-Stellung" des Gelenkes zur Folge hat, wenn nicht die Vorspannung des zweiten elastischen Elementes ebenfalls entsprechend angepasst wird. Zudem sind Gelenke bekannt, bei denen eine Anlagefläche des zweiten Gelenkteils verändert wird, um die "Null-Stellung" und damit die relative Position des zweiten Elementes relativ zum ersten Element zu verändern. Nachteilig ist hier, dass dazu das Gelenk dazu vollständig demontiert werden muss, da am zweiten Element, dessen Anlagefläche verändert wird, bauliche Veränderungen vorgenommen werden müssen, die nur bei demontiertem Gelenk möglich sind.

Der Erfindung liegt die Aufgabe zugrunde, ein Gelenk für eine orthopädietechnische Einrichtung so weiter zu entwickeln, dass diese Anpassung und Einstellung einfacher möglich ist.

Die Erfindung löst die gestellte Aufgabe durch ein Gelenk für eine orthopädietechnische Einrichtung gemäß dem Oberbegriff des Anspruchs 1, das sich dadurch auszeichnet, dass der Träger ein Anlageelement aufweist, das bei Erreichen des ersten Eingriffswinkels an dem zweiten Element anliegt, wobei das Anlageelement bei montierten Gelenk derart verstellbar ist, so dass der erste Eingriffswinkel und eine Vorspannung des elastischen Elementes unabhängig voneinander einstellbar sind.

Durch diese Ausgestaltung ist es nicht mehr notwendig, zur Veränderung des ersten Eingriffswinkels das Gelenk zu demontieren. Selbst eine Demontage des Trägers vom ersten Element ist nicht mehr notwendig. Dies bedeutet, dass eine orthopädietechnische Einrichtung, beispielsweise eine Orthese oder Prothese auch im getragenen Zustand einfach eingestellt werden kann. Bei einer besonders einfachen Ausgestaltung lässt sich dies sogar durch den Träger der Orthese oder Prothese erreichen, so dass kein Orthopädietechniker mehr notwendig ist. Dies ist insbesondere dann von Vorteil, wenn der Träger der Orthese oder Prothese oder einer anderen orthopädietechnischen Einrichtung beispielsweise mehrere Paar Schuhe besitzt, die unterschiedliche Absatzhöhen aufweisen.

In einer bevorzugten Ausgestaltung des Gelenkes weist der Träger einen Einstellkanal auf, durch den ein Verstellelement des Trägers zum Verstellen des Anlageelementes erreichbar ist. Das Verstellelement ist vorteilhafterweise ein Formschlusselement, insbesondere ein Innensechskant. Selbstverständlich sind auch andere Formschlusselemente möglich.

Mit einem entsprechenden Werkzeug, beispielsweise einem Inbusschlüssel oder einem anderen Formschlussgegenelement lässt sich folglich auch im montierten Zustand das Anlageelement verstellen, indem das entsprechende Werkzeug in den Einstellkanal eingeführt und beispielsweise an dessen dem Eingang abgewandten Ende in das Formschlusselement des Verstellelementes eingebracht wird. Durch den dabei erreichten Formschluss kann durch das Werkzeug das Verstellelement verstellt und somit das Anlageelement eingerichtet werden.

Vorzugsweise verfügt der Träger über ein Vorspannelement, durch das eine Vorspannung des wenigstens einen elastischen Elementes einstellbar ist, wobei das Vorspannelement vorzugsweise ein Formschlusselement, besonders bevorzugt ein Innensechskant ist.

Durch die Vorspannung des elastischen Elementes lässt sich einerseits die Größe der durch das elastische Element aufgebrachten Kraft einstellen. Auf diese Weise lässt sich folglich die Dämpfung einstellen, mit der der Anschlag, der die maximale Verschwenkbarkeit des ersten Elementes relativ zum zweiten Element bildet, erreicht wird. Ist das elastische Element beispielsweise als Schraubenfeder oder Schraubentellerfeder oder als Stapel von Tellerfedern ausgebildet, verfügt der Träger über zwei Federanlageelemente, zwischen denen das elastische Element angeordnet ist. In diesem Fall lässt sich besonders einfach die Vorspannung einstellen, indem diese beide Federanlageelemente aufeinander zu oder voneinander weg bewegt werden, um die Vorspannung in der Feder zu erhöhen oder abzusenken. Dies kann auf besonders einfache Weise technisch realisiert werden, indem eines der beiden Federanlageelemente an einem beispielsweise in einen Grundkörper des Trägers einschraubbaren Element angeordnet sind. Das Element kann in den Grundkörper des Trägers eingeschraubt oder aus ihm herausgeschraubt werden, so dass das sich an ihm befindende Federanlageelement auf das jeweils andere Federanlageelement zu- oder von diesem wegbewegt wird.

Dies geschieht vorzugsweise durch ein Formschlusselement, in das ein entsprechendes Werkzeug eingesetzt werden kann, und das auf diese Weise hinein- oder herausgeschraubt wird.

Vorzugsweise verläuft der Einstellkanal durch dieses Vorspannelement hindurch. Insbesondere für den Fall, dass das Vorspannelement selbst ein Formschlusselement ist, hat es sich daher als vorteilhaft herausgestellt, wenn ein Durchmesser des Formschlusselementes, das das Vorspannelement bildet, größer ist als ein Durchmesser des Formschlusselementes, das das Verstellelement bildet.

Vorteilhafterweise ist der Träger in ein dafür an dem ersten Element vorhandenes Gewinde anschraubbar oder aus ihm entfernbar, ohne dass eine eingestellte Vorspannung und/oder ein eingestellter erster Eingriffswinkel geändert wird. Hierzu kann ein weiteres Formschlusselement vorhanden sein, das in dem Einstellkanal enthalten ist und vorzugsweise einen Durchmesser aufweist, der zwischen dem Formschlusselement, das das Vorspannelement bildet und dem Durchmesser des Formschlusselementes, das das Verstellelement bildet liegt.

Der Einstellkanal kann in diesem Fall als Art Stufenkanal ausgebildet sein, dessen Durchmesser von seiner Öffnung, die vorzugsweise dem zweiten Element des Gelenks abgewandt ist, zum gegenüberliegenden Ende hin stufenförmig abnimmt. An jeder Stufe ist ein Formschlusselement vorhanden, in das ein entsprechendes Werkzeug mit einem korrespondierend ausgebildeten Formschlussgegenelement eingesetzt und betätigt werden kann, um eine bestimmte Funktion zu erfüllen. In einer besonders bevorzugten Ausführungsform befindet sich am Eingang des Einstellkanals das größte Formschlusselement. Dieses ist vorzugweise verwendbar, um die Vorspannung des elastischen Elementes einzustellen. Dringt ein Werkzeug tiefer in den Einstellkanal ein, ist es offenbar nicht geeignet, mit diesem Formschlusselement, dass das Vorspannelement bildet, zusammenzuwirken. Es kann beispielsweise in das nächst kleinere Formschlusselement eingreifen, das beispielsweise verwendet werden kann, um den Träger in das dafür vorgesehene und vorhandene Gewinde einzuschrauben. Ein noch kleineres Werkzeug, dessen Durchmesser des Formschlussgegenelementes auch zu klein ist um mit diesem zweiten Formschlusselement auf den Weg in den Einstellkanal zusammenzuwirken kann beispielsweise ein Formschlussgegenelement für ein Formschlusselement aufweisen, dass das Verstellelement bildet. Das Werkzeug mit dem Formschlussgegenelement greift folglich in das Formschlusselement, das das Verstellelement bildet, ein und kann auf diese Weise verwendet werden, um das Anlageelement zu verschieben und somit den ersten Eingriffswinkel einzustellen.

In einer bevorzugten Ausgestaltung verfügt der Träger über einen Grundkörper, der an dem ersten Element montierbar oder montiert ist, und über einen Schieber, der relativ zu dem Grundkörper verschiebbar ist und an dem sich das Anlageelement befindet. Der Schieber weist zudem vorzugsweise ein Federanlageelement auf, sodass eine Verschiebung des Schiebers relativ zum Grundkörper des Trägers eine Kompression oder eine Entspannung der Feder zur Folge hat.

Vorteilhafterweise verfügt der Schieber über einen Anschlag, der bei Erreichen einer maximalen Verschiebung relativ zu dem Grundkörper an einer Anschlagsfläche am Grundkörper anliegt. Auf diese Weise wird verhindert, dass der Anschlag, der eine maximale Verschwenkung des Gelenkes bildet, dadurch erreicht wird, dass eine Schraubenfeder oder eine Schraubentellerfeder auf Block gefahren wird. Bevorzugt ist dieser Block verstellbar.

Vorzugsweise ist die maximale Verschiebung einstellbar. Dies geschieht vorzugsweise indem der Schieber relativ zu dem Grundkörper und/oder der Anschlag relativ zu dem Schieber verschoben wird.

In einer bevorzugten Ausgestaltung verfügt ein derartiges Gelenk über zwei Träger mit jeweils wenigstens einem elastischen Element, durch die Kräfte in unterschiedlichen Richtungen auf das zweite Element aufbringbar sind. Ein derartiges Gelenk kann beispielsweise in eine Knöchel- oder Beinorthese oder Prothese eingesetzt werden und so beispielsweise eine Kraft sowohl in Plantarflexionsrichtung als auch in Dorsalflexionsrichtung aufbringen. Über die Wahl unterschiedlicher Federn oder unterschiedlicher Einstellungen der Vorspannung der unterschiedlichen Feder können auch unterschiedliche Kräfte für beide Bewegungsrichtungen realisiert werden.

Die Erfindung löst die gestellte Aufgabe zudem durch einen Träger für ein derartiges hier beschriebenes Gelenk.

Mit Hilfe der beiliegenden Figuren wird nachfolgend ein Ausführungsbeispiel der vorliegenden Erfindung näher erläutert. Es zeigt
Figur 1a und 1b - zwei Schnittdarstellungen durch einen Träger gemäß einem Ausführungsbeispiel der vorliegenden Erfindung in zwei unterschiedlichen Schnittebenen,
Figur 2 - eine schematische Seitenansicht und eine Schnittdarstellung durch ein Gelenk gemäß einem ersten Ausführungsbeispiel der vorliegenden Erfindung,
Figur 3 - die Darstellungen aus Figur 2 in einer anderen Stellung des Gelenkes,
Figur 4 - die Darstellungen aus Figur 2 für ein Gelenk gemäß einem weiteren Ausführungsbeispiel,
Figur 5 - die schmatischen Schnittdarstellungen durch ein Gelenk gemäß einem weiteren Ausführungsbeispiel der vorliegenden Erfindung,
Figuren 6 und 7 - schematische Schnittdarstellungen durch das Gelenk aus Figur 5 und
Figur 8 - schematische Schnittdarstellungen durch einen Träger in unterschiedlichen Positionen.

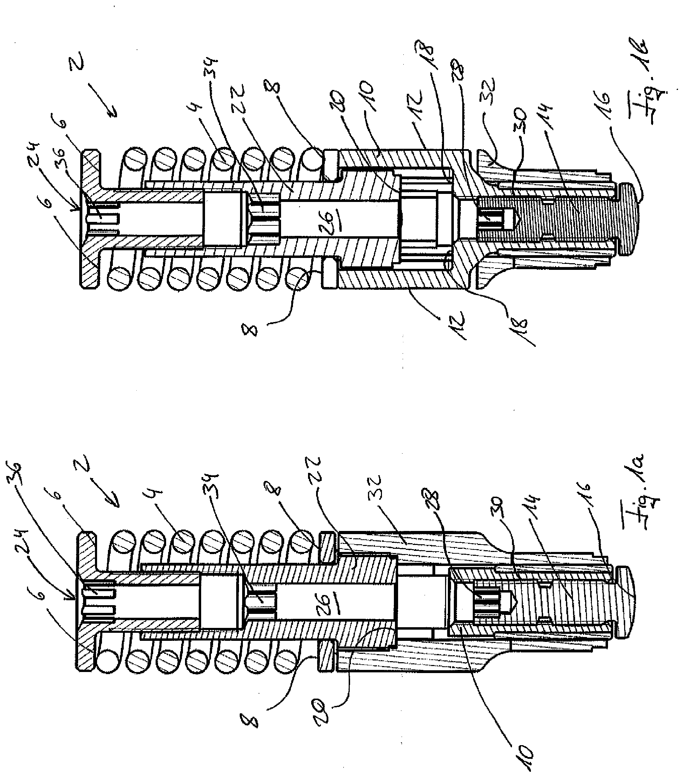

Die Figuren 1a und 1b zeigen einen Träger 2 in zwei Schnittdarstellungen, wobei der Träger 2 in den Figuren 1a und 1b relativ zueinander um 90° um seine Längsachse, die in den Figuren 1a und 1b von oben nach unten verläuft, gedreht ist. Am Träger 2 ist jeweils ein elastisches Element 4 angeordnet, das vorliegend als Schraubenfeder ausgebildet ist. Das elastische Element liegt an oberen Federanlageflächen 6 und unteren Federanlageflächen 8 an, wobei die unteren Federanlageflächen 8 an einem Schieber 10 angeordnet sind. Dieser Schieber 10 ist in Figur 1a in seinem unteren Anteil geschnitten und darüber in einer seitlichen Ansicht dargestellt und in Figur 1b dargestellt. Er verfügt im gezeigten Ausführungsbeispiel über zwei Schenkel 12, die um 180° versetzt zueinander angeordnet.

Der Schieber 10 ist mit einem Stößel 14 verbunden, an dem sich ein Anlageelement 16 befindet. Wird auf das Anlageelement 16 ein Druck in Figur 1a und 1b nach oben ausgeübt, wird dieser Druck über den Schieber 10 und dessen Schenkel 12 auf die untere Federauflagefläche 8 übertragen, wodurch das elastische Element 4 komprimiert wird.

Der Schieber 10 verfügt dabei über einen Anschlag 18, der bei Erreichen einer maximalen Verschiebung an einer Anschlagsfläche 20 eines Grundkörpers 22 anliegt und so die maximale Verschwenkung definiert. Der Grundkörper 22 wird auch Federdom genannt.

Der Träger 2 verfügt über eine Öffnung 24 zu einem Einstellkanal 26, der für unterschiedliche Funktionen verwendet wird. Der Einstellkanal 26 verfügt in den Figuren 1a über einen von oben nach unten abnehmenden Querschnitt. Im unteren Bereich befindet sich ein Einstellelement 28, das im gezeigten Ausführungsbeispiel als Formschluss ausgebildet ist. Ein Werkzeug, das durch die Öffnung 24 in den Einstellkanal 26 eingeführt wird und ein passendes Formschlussgegenelement aufweist, kann mit dem Einstellelement 28 zusammenwirken. Wird dieses Werkzeug gedreht, kann das Anlageelement 16, das über ein Gewinde 30 am Schieber 10 gelagert ist, aus dem Schieber 10 heraus oder in ihn hineingedreht werden. Dadurch wird der erste Eingriffswinkel angepasst.

Der Träger 2 ist über ein Befestigungselement 32 in ein nicht dargestelltes Bauteil eines Gelenkes einschraubbar.

Ein Werkzeug, das an seinem Ende ein anderes Formschlussgegenelement aufweist, kann mit einem Formschlusselement 34 zusammenwirken, dass mit dem Grundkörper 22 des Trägers 2 verbunden ist. Ein Werkzeug mit dem passenden Formschlussgegenelement, das mit dem Formschlusselement 34 in Eingriff gebracht wird, kann verwendet werden, um den Grundkörper 22 relativ zum Befestigungselement 32 zu verschieben. Auf diese Weise kann der Abstand zwischen der Anschlagsfläche 20 und dem Anschlag 18 eingestellt und somit der maximale Verschiebeweg bestimmt werden.

Im Bereich der Öffnung 24 verfügt der Träger 2 über ein Vorspannelement 36, das ebenfalls als Formschlusselement ausgebildet ist. Ein Werkzeug mit einem passend ausgebildeten Formschlussgegenelement kann mit dem Vorspannelement 36 in Eingriff gebracht werden und so das Bauteil mit den oberen Federanlageflächen 6 in den Grundkörper 22 hinein oder aus ihm heraus bewegen. Auf diese Weise kann die Vorspannung des Elastischen Elementes 4 verändert werden.

Soll nun der Träger beispielsweise aus einem Gelenk ausgebaut und in ein anderes Gelenk eingesetzt werden, muss lediglich das Befestigungselement 32 aus der dafür vorgesehenen Gewindeöffnung des Elementes des Gelenkes herausgeschraubt werden. Auf diese Weise kann der gesamte Träger aus dem Gelenk entfernt werden, ohne das eine Einstellung der Vorspannung über das Vorspannelement 36, einer Einstellung des maximalen Verschiebeweges über das Formschlusselement 34 oder eine Einstellung des ersten Eingriffswinkels über das Anlageelement 16 verändert werden muss. Dennoch ist es möglich, auch im eingesetzten Zustand alle diese Größen einzustellen, ohne den Träger 2 ausbauen zu müssen.

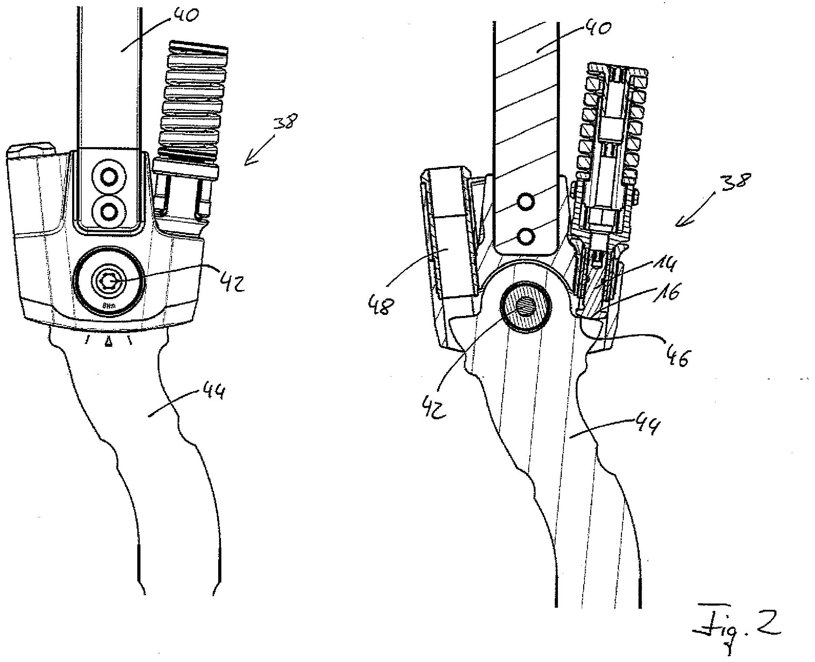

Figur 2 zeigt im linken Bereich eine schematische 3D-Ansicht eines Gelenkes 38 gemäß einem Ausführungsbeispiel der vorliegenden Erfindung. Im rechten Teil der Figur 2 ist die gleiche Darstellung als Schnittdarstellung gezeigt. Das Gelenk 38 verfügt über ein erstes Element 40 und ein um eine Schwenkachse 42 darin angeordnetes zweites Element 44. Man erkennt im rechten Teil der Figur 2, dass das Anlageelement 16 am Stößel 14 an einer Schulter 46 des zweiten Elementes 44 anliegt. Das elastische Element 4 ist nicht vollständig gespannt, sodass eine Bewegung des zweiten Elementes 44 um die Schwenkachse 42 in beide Richtungen möglich ist. Das erste Element 30 weist einen weiteren Gewindeeinsatz 48 auf, in den ein weiterer Träger 2 eingesetzt werden kann.

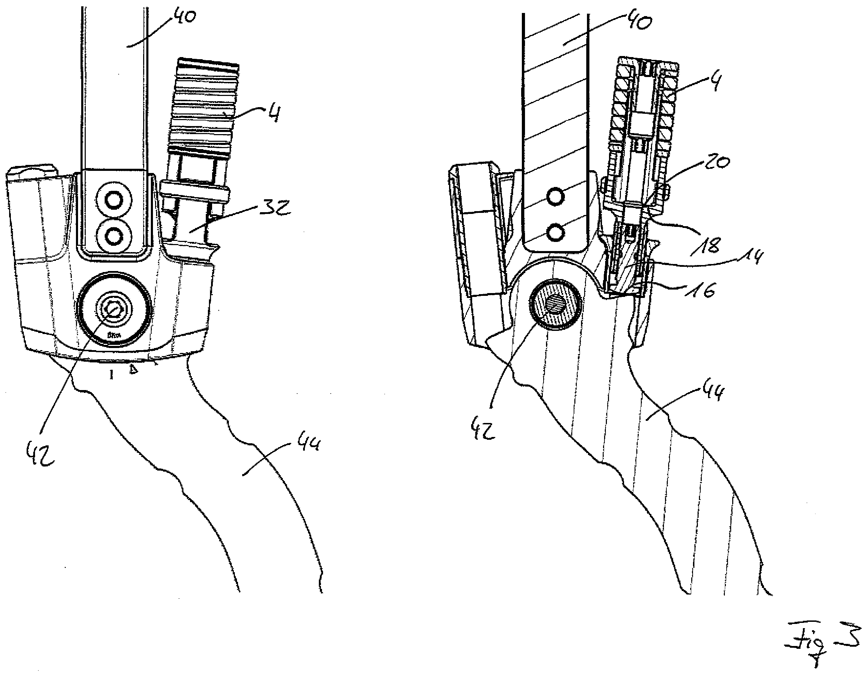

Figur 3 zeigt die beiden Darstellungen aus Figur 2, wobei nun das zweite Element 44 um die Schwenkachse 42 gegen das erste Element 40 verschwenkt ist. Dabei wird das elastische Element 4 stark komprimiert, sodass es eine Kraft über die in den Figuren 1a und 1b gezeigten unteren Federanlageflächen 8 auf den Schieber 10 und damit auf den Stößel 14 und das Anlageelement 16 ausübt. Man erkennt im rechten Teil der Figur 3, dass der Anschlag 18 an der Anschlagsfläche 20 anliegt. Der maximale Verschiebeweg ist somit erreicht. Zudem erkennt man, dass das Anlageelement 16, das am Stößel 14 angeordnet ist, im Vergleich zur Darstellung in Figur 3 nach oben verschoben worden ist.

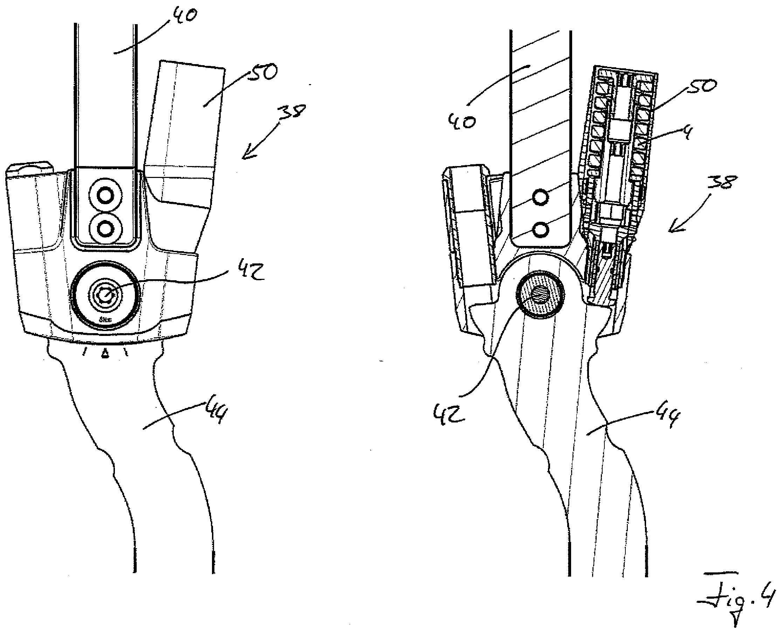

Figur 4 zeigt die Darstellung aus Figur 2 für ein Gelenk 38 gemäß einem anderen Ausführungsbeispiel der vorliegenden Erfindung. Der Hauptunterschied besteht darin, dass eine Hülse 50 über den Träger 2 gestülpt ist, sodass das elastische Element 4 nicht mehr von außen sichtbar ist. Dadurch wird die Verschmutzungsgefahr für das elastische Element 4 reduziert und gleichzeitig das Verletzungsrisiko für beispielsweise den Träger einer Orthese, die mit dem Gelenk ausgerüstet ist, reduziert.

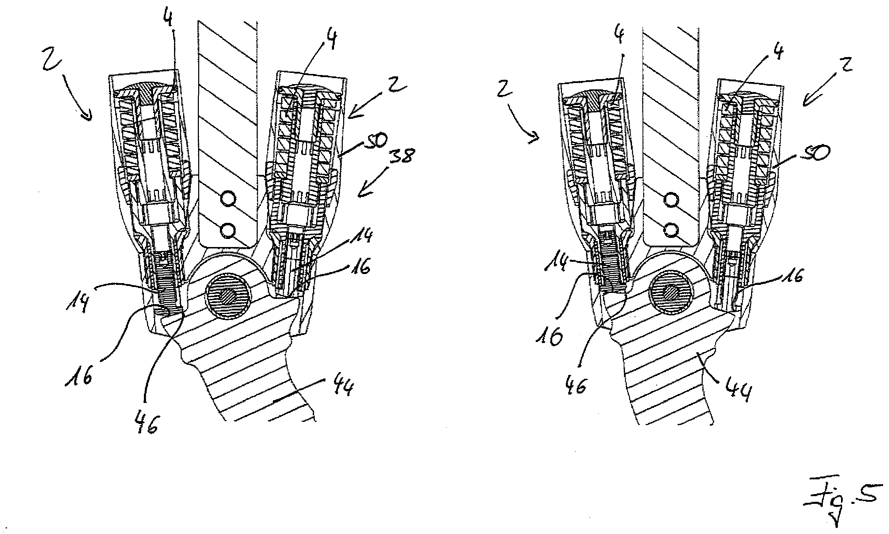

Figur 5 zeigt ein weitere Schnittdarstellungen durch ein Gelenk 38, das nun über zwei Träger 2 verfügt. Sie sind gleich aufgebaut und entsprechen im Wesentlichen den in den Figuren 1a und 1b gezeigten Ausführungsformen, wobei wiederrum eine Hülse 50 über den jeweiligen Träger 2 gestülpt wurde. Das Gelenk 38 befindet sich in beiden Darstellungen in der Nullstellung oder Ruheposition, sodass auf beiden Seiten jeweils der erste Eingriffswinkel gezeigt ist. Diese sind so gewählt, dass das Gelenk in der jeweils gezeigten Position diese Winkel für beide Seiten gleichzeitig einnimmt. Vergleicht man beide Darstellungen der Figur 5, so erkennt man, dass jeweils der Anschlag und der erste Eingriffswinkel geändert wurde, ohne dass dazu eines der elastischen Elemente in seiner Vorspannung verändert werden musste.

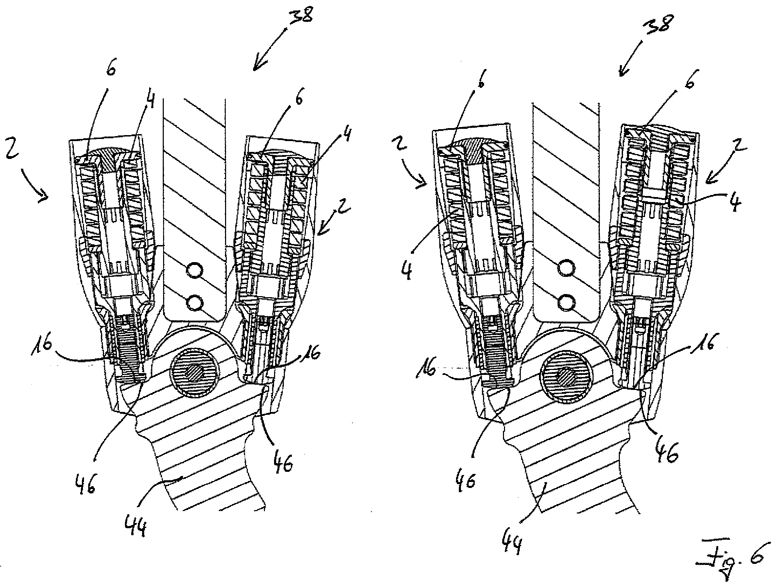

Figur 6 zeigt zwei Darstellungen des Gelenks 38 mit jeweils zwei Trägern 2, wobei wiederrum jeweils ein Anlageelement 16 an den beiden Schultern 46 des zweiten Elementes 44 anliegt. Auch hier ist die Position gezeigt, in der die ersten Eingriffswinkel auf beiden Seiten gleichzeitig eingenommen werden. Der Vergleich der beiden Darstellungen in Figur 6 macht jedoch deutlich, dass eines der beiden Bauelemente mit den oberen Federanlageflächen 6 in der rechten Darstellung in Figur 6 bei beiden Trägern 2 deutlich weiter nach innen geschraubt wurde, als dies in der linken Darstellung der Fall ist. Dadurch wird die Vorspannung des elastischen Elementes 4 vergrößert, ohne dass der erste Eingriffswinkel verändert wird.

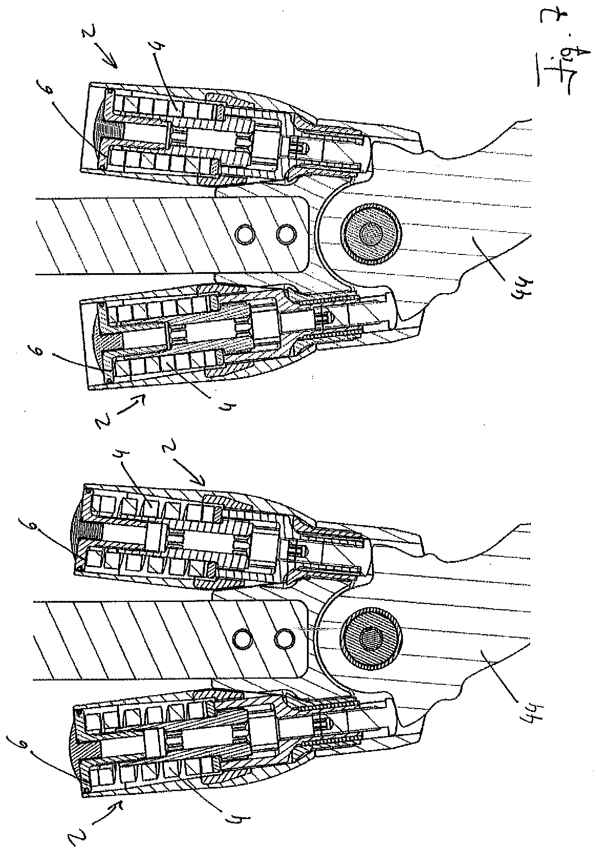

Gleiches gilt für Figur 7. Es zeigt die beiden Darstellungen aus Figur 6 jedoch in einer anderen Gleichgewichtslage. Auch hier ist jeweils das Element mit den oberen Federanlageflächen 6 in der rechten Darstellung deutlich weiter nach innen geschraubt, als dies in der linken Darstellung der Fall ist, wodurch die Vorspannung der jeweiligen elastischen Elemente 4 in der rechten Darstellung im Vergleich zur linken Darstellung stark angestiegen ist.

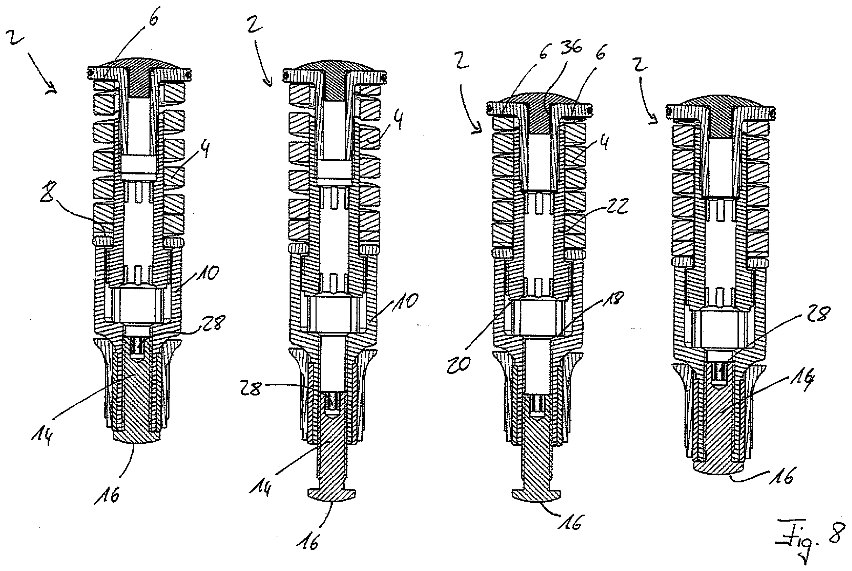

Figur 8 zeigt den Träger 2 in vier unterschiedlichen Positionen. Ganz links ist eine Ausgangsposition gezeigt, in der der Stößel 14 mit dem daran befindlichen Anlageelement 16, die obere Federanlagefläche 6, die untere Federanlagefläche 8 und das elastische Element 4 dargestellt ist. Die zweite Darstellung von links wurde im Vergleich zur ganz linken Darstellung dahingehend verändert, dass der Stößel 14 und mit ihm das daran angeordnete Anlageelement 16 relativ zum Schieber 10 verlagert wurde, indem ein entsprechendes Werkzeug mit dem Einstellelement 28 in Eingriff gebracht und entsprechend betätigt wurde. Weder der maximale Verschiebeweg noch die Vorspannung des elastischen Elementes 4 wurden dabei verändert.

In der zweiten Darstellung von rechts wurde im Vergleich zur zweiten Darstellung von links die Vorspannung des elastischen Elementes 4 verändert, in dem das Bauteil mit den oberen Federanlageflächen 6 relativ zum Grundkörper 22 verlagert wurde, indem ein entsprechendes Werkzeug mit dem Vorspannelement 36 in Eingriff gebracht wurde. Dadurch wurde weder der maximal Verschiebeweg, der durch den Abstand von Anschlagsfläche 20 und Anschlag 18 bestimmt ist, noch die Position des ersten Eingriffswinkels, der durch die Position des Anlageelementes 16 bestimmt ist, verändert.

In der ganz rechten Darstellung der Figur 8 wurde im Vergleich zur zweiten Darstellung von rechts der Stößel 14 und damit das Anlageelement 16 wieder nach oben verschoben, in dem wieder ein Werkzeug mit dem Einstellelement 28 in Eingriff gebracht wurde.

### Bezugszeichenliste:

- 2: Träger
- 4: Elastisches Element
- 6: obere Federanlagefläche
- 8: untere Federanlagefläche
- 10: Schieber
- 12: Schenkel
- 14: Stößel
- 16: Anlageelement
- 18: Anschlag
- 20: Anschlagsfläche

- 22: Grundkörper
- 24: Öffnung
- 26: Einstellkanal
- 28: Einstellelement
- 30: Gewinde

- 32: Befestigungselement
- 34: Formschlusselement
- 36: Vorspannelement
- 38: Gelenk
- 40: erstes Element

- 42: Schwenkachse
- 44: zweites Element
- 46: Schulter
- 48: Gewindeeinsatz
- 50: Hülse

- 52: gestrichelte Linie

## Patentansprüche

1. Gelenk (38) für eine orthopädietechnische Einrichtung, wobei das Gelenk (38)
- ein erstes Element (40),
- wenigstens ein elastisches Element (4) an einem Träger (2), der an dem ersten Element (40) montiert ist, und
- ein zweites Element (44) aufweist, das an dem ersten Element (40) schwenkbar angeordnet ist und in einer Richtung ab einem ersten Eingriffswinkel zwischen dem ersten Element (40) und dem zweiten Element (44) gegen eine von dem wenigstens einen elastischen Element (4) aufgebrachte Kraft schwenkbar ist,
**dadurch gekennzeichnet, dass**
der Träger (2) ein Anlageelement (16) aufweist, das bei Erreichen des ersten Eingriffswinkels an dem zweiten Element (44) anliegt, wobei das Anlageelement (16) bei montiertem Gelenk (38) derart verstellbar ist, so dass der erste Eingriffswinkel und eine Vorspannung des elastischen Elementes (4) unabhängig voneinander einstellbar sind.

2. Gelenk (38) nach Anspruch 1, **dadurch gekennzeichnet, dass** der Träger (2) einen Einstellkanal (26) aufweist, durch den ein Verstellelement (28) des Trägers (2) zum Verstellen des Anlageelementes (16) erreichbar ist.

3. Gelenk (38) nach Anspruch 1 oder 2, **dadurch gekennzeichnet, dass** das Verstellelement (28) ein Formschlusselement, insbesondere ein Innensechskant ist.

4. Gelenk (38) nach einem der vorstehenden Ansprüche, **dadurch gekennzeichnet, dass** der Träger (2) ein Vorspannelement (36) aufweist, durch das eine Vorspannung des wenigstens einen elastischen Elementes (4) einstellbar ist, wobei das Vorspannelement (36) vorzugsweise ein Formschlusselement, besonders bevorzugt ein Innensechskant ist.

5. Gelenk (38) nach Anspruch 4, **dadurch gekennzeichnet, dass** der Einstellkanal (26) durch das Vorspannelement (36) hindurch verläuft.

6. Gelenk (38) insbesondere nach einem der vorstehenden Ansprüche, **dadurch gekennzeichnet, dass** der Träger (2) in ein dafür an dem ersten Element (40) vorhandenes Gewinde einschraubbar oder aus ihm entfernbar ist, ohne dass eine eingestellte Vorspannung und/oder ein eingestellter erster Eingriffswinkel geändert wird.

7. Gelenk (38) nach Anspruch 6, **dadurch gekennzeichnet, dass** der Träger (2) einen Grundkörper (22), der an dem ersten Element (40) montierbar oder montiert ist, und einen Schieber (10) aufweist, der relativ zu dem Grundkörper (22) verschiebbar ist und an dem das Anlageelement (16) befindet angeordnet ist.

8. Gelenk (38) nach Anspruch 7, **dadurch gekennzeichnet, dass** der Schieber (10) einen Anschlag (18) aufweist, der bei Erreichen einer maximalen Verschiebung relativ zu dem Grundkörper (22) an einer Anschlagsfläche (20) am Grundkörper (22) anliegt.

9. Gelenk (38) nach Anspruch 8, **dadurch gekennzeichnet, dass** die maximale Verschiebung einstellbar ist, vorzugsweise indem der Schieber (10) relativ zu dem Grundkörper (22) und/oder der Anschlag (18) relativ zu dem Schieber (10) verschoben wird.

10. Gelenk (38) nach einem der vorstehenden Ansprüche, **dadurch gekennzeichnet, dass** das Gelenk (38) zwei Träger (2) mit jeweils wenigstens einem elastischen Element (4) aufweist, durch die Kräfte in unterschiedlichen Richtungen auf das zweite Element (44) aufbringbar sind.

## Claims

1. A joint (38) for an orthopedic device, wherein the joint (38) comprises
- a first element (40),
- at least one elastic element (4) on a support (2), which is mounted on the first element (40), and
- a second element (44) which is pivotally arranged on the first element (40) and can be swivelled in a direction against a force applied by the at least one elastic element (4) starting from a first angle of engagement between the first element (40) and the second element (44),
**characterized by the fact that**
the support (2) comprises a contact element (16) which rests against the second element (44) upon reaching the first angle of engagement, wherein the contact element (16) can be adjusted when the joint (38) is assembled such that the first angle of engagement and a preload of the elastic element (4) can be adjusted independently of one another.

2. The joint (38) according to claim 1, **characterized by the fact that** the support (2) features an adjustment channel (26), by way of which an adjustment element (28) of the support (2) can be reached in order to adjust the contact element (16).

3. The joint (38) according to claim 1 or 2, **characterized by the fact that** the adjustment element (28) is a positive-locking element, especially a hexagon socket.

4. The joint (38) according to one of the above claims, **characterized by the fact that** the support (2) comprises a pre-loading element (36), by way of which a preload of the at least one elastic element (4) can be adjusted, wherein the pre-loading element (36) is preferably a positive-locking element, especially preferably a hexagon socket.

5. The joint (38) according to claim 4, **characterized by the fact that** the adjustment channel (26) extends through the pre-loading element (36).

6. The joint (38), in particular according to one of the preceding claims, **characterized by the fact that** the support (2) can screwed into or removed from a specially provided thread on the first element (40), without having to alter a set preload and/or a set first angle of engagement.

7. The joint (38) according to claim 6, **characterized by the fact that** the support (2) has a base body (22), which can be or is assembled on the first element (40), and a slide (10), which can be displaced relative to the base body (22), wherein the contact element (16) is arranged on the slide (10).

8. The joint (38) according to claim 7, **characterized by the fact that** the slide (10) comprises an end stop (18), which rests on an end stop surface (20) on the base body (22) upon reaching a maximum displacement relative to the base body (22).

9. The joint (38) according to claim 8, **characterized by the fact that** the maximum displacement can be adjusted, preferably by displacing the slide (10) relative to the base body (22) and/or the end stop (18) relative to the slide (10).

10. The joint (38) according to one of the above claims, **characterized by the fact that** the joint (38) features two supports (2), each of which has at least one elastic element (4), by way of which the forces can be applied to the second element (44) in different directions.

## Revendications

1. Articulation (38) pour un dispositif orthopédique, l'articulation (38) comprenant
- un premier élément (40),
- au moins un élément élastique (4) qui est disposé sur un support (2) monté sur le premier élément (40), et
- un deuxième élément (44) qui est disposé de manière pivotante sur le premier élément (40) et qui peut pivoter à l'encontre d'une force, exercée par ledit au moins un élément élastique (4), dans une direction à partir d'un premier angle d'engagement entre le premier élément (40) et le deuxième élément (44),
**caractérisée en ce que**
le support (2) comprend un élément d'appui (16) qui, lorsque le premier angle d'engagement est atteint, s'appuie contre le deuxième élément (44), et l'articulation (38) étant montée, l'élément d'appui (16) peut être réglé de telle sorte que le premier angle d'engagement et une précontrainte de l'élément élastique (4) peuvent être ajustés indépendamment l'un de l'autre.

2. Articulation (38) selon la revendication 1,
**caractérisée en ce que** le support (2) présente un canal d'ajustement (26) à travers lequel un élément de réglage (28) du support (2) est accessible pour régler l'élément d'appui (16).

3. Articulation (38) selon la revendication 1 ou 2,
**caractérisée en ce que** l'élément de réglage (28) est un élément à liaison par complémentarité de forme, en particulier un six pans creux.

4. Articulation (38) selon l'une des revendications précédentes,
**caractérisée en ce que** le support (2) comprend un élément de précontrainte (36) permettant d'ajuster une précontrainte dudit au moins un élément élastique (4), l'élément de précontrainte (36) étant de préférence un élément à liaison par complémentarité de forme, de manière particulièrement préférée un six pans creux.

5. Articulation (38) selon la revendication 4,
**caractérisée en ce que** le canal d'ajustement (26) s'étend à travers l'élément de précontrainte (36).

6. Articulation (38) selon l'une des revendications précédentes, **caractérisée en ce que** le support (2) peut être vissé dans un filetage, prévu à cet effet sur le premier élément (40), ou être retiré de celui-ci, sans modifier une précontrainte ajustée et/ou un premier angle d'engagement ajusté.

7. Articulation (38) selon la revendication 6,
**caractérisée en ce que** le support (2) comprend un corps de base (22) qui peut être monté ou est monté sur le premier élément (40), et un coulisseau (10) qui peut coulisser par rapport au corps de base (22) et sur lequel est disposé l'élément d'appui (16).

8. Articulation (38) selon la revendication 7,
**caractérisée en ce que** le coulisseau (10) comprend une butée (18) qui, lorsqu'une course de coulissement maximale par rapport au corps de base (22) est atteinte, vient en appui contre une surface de butée (20) sur le corps de base (22).

9. Articulation (38) selon la revendication 8,
**caractérisée en ce que** la course de coulissement maximale est ajustable, de préférence du fait que le coulisseau (10) est déplacé par rapport au corps de base (22) et/ou que la butée (18) est déplacée par rapport au coulisseau (10).

10. Articulation (38) selon l'une des revendications précédentes,
**caractérisée en ce que** l'articulation (38) comprend deux supports (2) munis chacun d'au moins un élément élastique (4), permettant d'appliquer des forces au deuxième élément (44) dans différentes directions.
